# EUROPEAN PATENT APPLICATION

(11) **EP 1 938 813 A1**
(43) Date of publication of application: **02.07.2008**
(21) Application number: 06782674.3
(22) Date of filing: 04.08.2006
(51) Int. Cl.: A61K 31/198, A61P 1/16, A61P 3/10, A61P 7/00, A61P 13/12

(54) **AGENT FOR REDUCTION OF OXIDIZED ALBUMIN LEVEL**

(30) Priority: 04.08.2005 JP 2005226967
(71) Applicant: Ajinomoto Co., Inc., Tokyo 104-8315 (JP)
(72) Inventor: MORIWAKI, Hisataka, Gifu; 501-1194 (JP); FUKUSHIMA, Hideki, Gifu 501-1194 (JP); ERA, Seiichi, Gifu 501-1194 (JP); TAKEHANA, Kenji c/o AJINOMOTO CO., INC., 1-1,, 2108 681 (JP); KAWAKAMI, Asami c/o AJINOMOTO CO., INC., 1-1,, 2108 681 (JP); TANABE, Itsuya c/o AJINOMOTO CO., INC., 1-1,, 21086 81 (JP); YAMADA, Naoyuki c/o AJINOMOTO CO., INC., 1-1,, 2108 681 (JP); KUBOTA, Kazuyuki c/o AJINOMOTO CO., INC., 1-1,, 210 8681 (JP)
(74) Representative: Nicholls, Kathryn Margaret
(86) International application number: PCT/JP2006/315888
(87) International publication number: WO 2007/015585

(57) **Abstract**

The present invention provides a pharmaceutical agent and the like that decreases the level of oxidized albumin. Specifically, the present invention provides an agent for decreasing oxidized albumin, which contains at least one kind of branched-chain amino acid selected from isoleucine, leucine and valine.

## Description

### Technical Field

The present invention relates to a pharmaceutical agent that decreases oxidized albumin level in the body and the like.

### Background Art

Serum albumin is the most abundant simple protein in the body, and has three physiological functions of maintaining plasma oncotic pressure, binding and transportation of various endogenous or exogenous substances, and an amino acid source. As a substance to be transported by serum albumin, hormones such as tryptophan, urine acid, glutathione, ATP, bilirubin, siloxane, testosterone and the like, metal ions such as copper, zinc and the like, vitamins such as vitamin B6, vitamin C and the like, fatty acids such as palmitic acid, oleic acid and the like, pharmaceutical agent such as antibiotics, aspirin, etc. and the like are widely known (ed. Akiharu Watanabe *Rinshouarubumingaku,* Medical Review Co., Ltd).

Serum albumin is present as a mixture of reduced albumin having a cysteine residue with a free SH group in position 34 from the N terminal, and oxidized albumin wherein the cysteine residue forms a disulfide (S-S) bond with cysteine, glutathione and the like in the blood. In normal human serum, about 75% is of a reduced type and about 25% is of an oxidized type. It has been reported that cirrhosis patients (Watanabe, A. Nutrition 20:351, 2004), chronic renal failure patients, diabetes patients and patients under anesthesia or operation show an increased proportion of oxidized albumin (Seiichi Era, Rinshou Kensa 48:501-511, 2004).

While an increase in the oxidized albumin is assumed to relate to the redox state in the body and influence the binding and transportation of substances, its pathological significance has not been elucidated. In addition, while a report (Sogami, M. Int. J. Peptide Protein Res 25: 398, 1985) has documented that hemodialysis decreases oxidized albumin in chronic renal failure patients and increases reduced albumin, a pharmaceutical agent or food component that decreases oxidized albumin and increases reduced albumin has not been known at all.

### Disclosure of the Invention

The problem to be solved by the present invention is to clarify the pathological significance of oxidized albumin and provide a pharmaceutical agent and the like that decrease the level of increased oxidized albumin.

The present inventors have conducted intensive studies in an attempt to solve the aforementioned problem and found that at least one kind of branched-chain amino acid selected from isoleucine, leucine and valine has an action to decrease the level of increased oxidized albumin, an action to improve an oxidized/reduced albumin ratio, and an action to improve a function of serum albumin and in vivo redox state, which resulted in the completion of the present invention. Accordingly, the present invention encompasses the following items.
(1) An agent for decreasing oxidized albumin, which comprises at least one kind of branched-chain amino acid selected from isoleucine, leucine and valine.
(2) An agent for decreasing oxidized albumin, which comprises three kinds of branched-chain amino acid of isoleucine, leucine and valine.
(3) The agent of the above-mentioned (2), wherein the weight ratio of isoleucine, leucine and valine is 1:1 - 3:0.5 - 2.0.
(4) The agent of the above-mentioned (2), wherein the weight ratio of isoleucine, leucine and valine is 1:1.5 - 2.5:0.8 - 1.5.
(5) The agent of any one of the above-mentioned (1) to (4), wherein a dose of the branched-chain amino acid is 1 g - 50 g in total per day for an adult.
(6) The agent of any one of the above-mentioned (1) to (4), wherein the dose of the branched-chain amino acid is 3 g - 30 g in total per day for an adult.
(7) The agent of any one of the above-mentioned (1) to (6), which is used for at least one kind of patient selected from patients with cirrhosis, chronic renal failure, diabetes, and under anesthesia or operation.
(8) An agent for improving oxidized/reduced albumin ratio, which comprises at least one kind of branched-chain amino acid selected from isoleucine, leucine and valine.
(9) An agent for improving a function of serum albumin, which comprises at least one kind of branched-chain amino acid selected from isoleucine, leucine and valine.
(10) The agent of the above-mentioned (9), wherein the function of serum albumin is at least one kind selected from tryptophan-binding ability, bilirubin-binding ability and fatty acid-binding ability.
(11) An agent for improving a redox state in the body, which comprises at least one kind of branched-chain amino acid selected from isoleucine, leucine and valine.
(12) A food or drink comprising at least one kind of branched-chain amino acid selected from isoleucine, leucine and valine, which indicates that the food or drink has an oxidized albumin lowering action.
(13) A food or drink comprising at least one kind of branched-chain amino acid selected from isoleucine, leucine and valine, which indicates that the food or drink has an in vivo redox state-improving effect.
(14) A method of decreasing oxidized albumin, which comprises administering an effective amount of at least one kind of branched-chain amino acid selected from isoleucine, leucine and valine to a subject.
(15) A method of improving an oxidized/reduced albumin ratio, which comprises administering an effective amount of at least one kind of branched-chain amino acid selected from isoleucine, leucine and valine to a subject.
(16) A method of improving a function of serum albumin, which comprises administering an effective amount of at least one kind of branched-chain amino acid selected from isoleucine, leucine and valine to a subject.
(17) A method of improving a redox state in the body, which comprises administering an effective amount of at least one kind of branched-chain amino acid selected from isoleucine, leucine and valine to a subject.
(18) Use of at least one kind of branched-chain amino acid selected from isoleucine, leucine and valine for the production of an agent for decreasing oxidized albumin.
(19) Use of at least one kind of branched-chain amino acid selected from isoleucine, leucine and valine for the production of an agent for improving an oxidized/reduced albumin ratio.
(20) Use of at least one kind of branched-chain amino acid selected from isoleucine, leucine and valine for the production of an agent for improving a function of serum albumin.
(21) Use of at least one kind of branched-chain amino acid selected from isoleucine, leucine and valine for the production of an agent for improving a redox state in the body.
(22) A commercial package comprising a pharmaceutical agent comprising at least one kind of branched-chain amino acid selected from isoleucine, leucine and valine and a pharmaceutically acceptable carrier, and a written matter describing that the pharmaceutical agent can or should be used for decreasing oxidized albumin.
(23) A commercial package comprising a pharmaceutical agent comprising at least one kind of branched-chain amino acid selected from isoleucine, leucine and valine and a pharmaceutically acceptable carrier, and a written matter describing that the pharmaceutical agent can or should be used for improving an oxidized/reduced albumin ratio.
(24) A commercial package comprising a pharmaceutical agent comprising at least one kind of branched-chain amino acid selected from isoleucine, leucine and valine and a pharmaceutically acceptable carrier, and a written matter describing that the pharmaceutical agent can or should be used for improving a function of serum albumin.
(25) A commercial package comprising a pharmaceutical agent comprising at least one kind of branched-chain amino acid selected from isoleucine, leucine and valine and a pharmaceutically acceptable carrier, and a written matter describing that the pharmaceutical agent can or should be used for improving a redox state in the body.
(26) A composition for decreasing oxidized albumin, which comprises at least one kind of branched-chain amino acid selected from isoleucine, leucine and valine.
(27) A composition for improving an oxidized/reduced albumin ratio, which comprises at least one kind of branched-chain amino acid selected from isoleucine, leucine and valine.
(28) A composition for improving a function of serum albumin, which comprises at least one kind of branched-chain amino acid selected from isoleucine, leucine and valine.
(29) A composition for improving a redox state in the body, which comprises at least one kind of branched-chain amino acid selected from isoleucine, leucine and valine.

### Brief Description of the Drawings

Fig. 1 is a graph showing the profile of the oxidized albumin ratio in Example 1.
Fig. 2 shows the oxidized albumin ratio in Example 2.
Fig. 3 shows the L-tryptophan binding rate in Example 2.
Fig. 4 is a chart showing the intensity of radical produced by each albumin in Example 3.
Fig. 5 shows the radical intensity relative to oxidized albumin ratio in Example 3.

### Best Mode for Embodying the Invention

The embodiment of the present invention is explained in the following.

The "agent for decreasing oxidized albumin" of the present invention means a pharmaceutical agent that decreases the ratio of oxidized albumin that increased due to a disease or other factors, in the ratio of reduced:oxidized serum albumin. That is, the agent of the present invention has an action to lower the increased level of oxidized albumin to a normal level, more specifically to 25% or near 25%, where the ratio of oxidized albumin and reduced albumin in a normal body is approximately 25% for the former and 75% for the latter.

The agent for decreasing oxidized albumin of the present invention is useful for patients associated with an increased ratio of oxidized albumin in the body. Specifically, it is useful for patients with cirrhosis, chronic renal failure, diabetes, under anesthesia or operation, and the like.

In addition, the agent for decreasing oxidized albumin of the present invention can be used as an agent for improving oxidized/reduced albumin ratio, an agent for improving a function of serum albumin and an agent for improving a redox state in the body.

As used herein, the oxidized/reduced albumin ratio means a ratio of oxidized and reduced serum albumin in the body, which value is considered to be normally about 2.5:7.5. When oxidized type increases due to hepatic diseases, diabetes, thyroid gland disease, nephrotic syndrome, renal failure, aging and the like, this ratio also varies. That is, an improvement of the oxidized/reduced albumin ratio means improving the ratio to a normal level, or close to the normal level.

The improving function of serum albumin means enhancing at least one function selected from tryptophan-binding ability, bilirubin-binding ability and fatty acid-binding ability that serum albumin has.

A normal tryptophan binding rate of serum albumin is considered to be not less than 90%. When this rate decreases markedly, the concentration of tryptophan present as a serum protein non-binding type in the blood increases, and an intracerebral serotonin conversion rate increases. This causes fatigability and encephalopathia.

A normal bilirubin binding rate of serum albumin is generally considered to be not less than 99%, and by the binding, unconjugated (indirect) bilirubin in the blood is transported to the liver. When the value decreases, the concentration of free bilirubin increases, and bilirubin moves into the brain to cause neurotoxicity.

In addition, a normal fatty acid (e.g., palmitic acid, oleic acid and the like) binding rate of serum albumin is generally considered to be not less than 99%. When the value decreases, the concentration of free fatty acid in the blood increases, causing toxicity such as hemolysis and the like.

Accordingly, improving a function of serum albumin means improving these numerical values to normal values, or close to the normal values.

The redox state in the body refers to a balanced state between oxidative reaction and antioxidative reaction (i.e., reduction reaction) in the body. In various pathologies, the balance collapses and the oxidation tendency occurs. As a result, oxidative stress is caused through production of active oxygen species such as active oxygen, free radical and the like, thereby damaging cells and tissues. Therefore, improvement of in vivo redox state means elimination of excess oxidative stress by activation of the anti-oxidative defense mechanism, thereby restoring the normal state, or close to the normal state.

Isoleucine, leucine and valine, which are the active ingredients (branched-chain amino acid) of the present invention, may be any of an L-form, a D-form and a DL-form. In addition, isoleucine, leucine and valine can be used not only in a free form but also as a salt or hydrate.

Examples of the salt form include acid addition salt, salts with base and the like. It is preferable to select a salt of isoleucine, leucine or valine, which is acceptable as a pharmaceutical product or a food or drink. Examples of the acid that is added to isoleucine, leucine or valine to form a salt acceptable as a pharmaceutical product or a food or drink include inorganic salts such as hydrogen chloride, hydrogen bromide, sulfuric acid, phosphate and the like, organic salts with acetic acid, lactic acid, citric acid, tartaric acid, maleic acid, fumaric acid or monomethylsulfuric acid and the like. Examples of the salt of isoleucine, leucine and valine with a base, which is acceptable as a pharmaceutical product or a food or drink, include hydroxide or carbonate of metal such as sodium, potassium, calcium and the like, a salt with an inorganic base such as ammonia and the like, and a salt with an organic base such as ethylenediamine, propylenediamine, ethanolamine, monoalkylethanolamine, dialkylethanolamine, diethanolamine, triethanolamine and the like. The above-mentioned salts may be hydrates (water-containing salts).

In addition, examples of the hydrate include 1 - 6 hydrate and the like. These salts and hydrates are also encompassed in the scope of the present invention.

The agent for decreasing oxidized albumin of the present invention only needs to contain at least any one kind of isoleucine, leucine and valine as a branched-chain amino acid. The agent preferably contains all of isoleucine, leucine and valine. More preferably, the weight ratio of isoleucine, leucine and valine is 1:1 - 3:0.5 - 2.0. Further preferably, the weight ratio of isoleucine, leucine and valine is 1:1.5 - 2.5:0.8 - 1.5. Most preferable weight ratio is 1:2:1.2. The administration method of the pharmaceutical agent of the present invention may be oral administration, or parenteral administration (intake) such as infusion, injection (intravenous administration) and the like and is not particularly limited. However, since the active ingredient is amino acid, oral administration is preferable.

While the dose (intake) of the agent for decreasing oxidized albumin of the present invention for oral administration varies depending on the symptom and age of the patient to be the administration subject, and administration method, it is generally 1 - 30 g of isoleucine, 1 - 30 g of leucine and 1 - 30 g of valine per day for one patient. For a general adult, the dose is preferably 2 - 10 g of isoleucine, 2 - 10 g of leucine and 2 - 10 g of valine, more preferably 2.5 - 3 g of isoleucine, 5 - 6 g of leucine and 3 - 4 g of valine, per day for an average adult. When all of isoleucine, leucine and valine are contained, the total amount of the dose per day for an adult is preferably 1 g - 50 g. More preferably, the total amount of the dose per day for an adult is 3 - 30 g, more preferably 4 - 25 g.

On the other hand, parenteral administration (intake) such as infusion administration, injection administration (intravenous administration) and the like is also possible. The dose in that case is about 1/10 - 1/20 of the preferable dose (intake) for the aforementioned oral administration.

The dosage forms of pharmaceutical preparation in general, such as granule, fine granule, tablet, powder, capsule, chewable preparation, liquid preparation, suspension and the like as an agent for oral administration, and intravenous direct injection, infusion administration and the like as an injection, can be employed.

Isoleucine, leucine and valine, which are the branched-chain amino acids of the present invention, may be each formulated singly into a preparation, or a combination of any two therefrom may be formulated into a preparation. Alternatively, a single preparation containing all of them may be produced. For administration of individual preparations, the administration route and administration dosage form thereof may be the same or different. In addition, the timing of administration thereof may be simultaneous or different.
They are appropriately determined in consideration of the kind and effect of the pharmaceutical agent to be used concurrently.

In the present invention, the "weight ratio" means the weight ratio of each component in a preparation. For example, when each of the branched-chain amino acids of isoleucine, leucine and valine is contained in a single preparation, the ratio is that of individual contents. When each or any combination of two of the branched-chain amino acids is contained in plural preparations, it is the ratio of the weight of each branched-chain amino acid contained in each preparation. In the present specification, the weight of each amino acid is that of a free amino acid.

The agent for decreasing oxidized albumin of the present invention can be formulated into a preparation by a conventional method. When necessary for formulation of a preparation, for example, a carrier such as lactose, glucose, D-mannitol, starch, crystalline cellulose, calcium carbonate, kaolin, gelatin and the like, a general additive such as solvent, solubilizing agent, isotonicity agent and the like can be appropriately combined.

The agent for decreasing oxidized albumin of the present invention can be particularly used as a pharmaceutical product or a food or drink (including health food, nutritional supplement and the like). Since the active ingredient is an amino acid, it is superior in the safety, and can be used conveniently in the form of a food or drink. It is also possible to provide such food and drink as a food with health claims. The food with health claims includes a food or drink with the indication that it has an oxidized albumin decreasing action, or that it has an in vivo redox state improving effect, particularly specified health food and the like. When it is used as a pharmaceutical product, its form is not particularly limited, and the above-mentioned preparation may be directly used. In the case of a food or drink, its form is not particularly limited, and only a branched-chain amino acid of an agent for decreasing oxidized albumin, or only an amount necessary for exhibiting the effect of the preparation may be contained in the food or drink.

The present invention is explained in more detail in the following by referring to Examples, which are not to be construed as limitative.

### Examples

### (Example 1)

### Consideration of oxidized albumin decreasing action by administration of branched-chain amino acid (BCAA) to noncompensated cirrhosis patient

The serum albumin of non-cirrhosis patients with hypoalbuminemia (serum albumin value not more than 3.5 g/dL) was examined for 8 weeks for the effect of administration of BCAA on the oxidized albumin ratio. The subject was patients (7 cases) with cirrhosis caused by HCV (hepatitis C virus) who were free of administration of an amino acid preparation for the last 4 weeks and had sufficient dietary intake. Patients under continuous administration of an albumin preparation or a freeze plasma preparation, patients with hepatocarcinoma, patients showing a total serum bilirubin value of not less than 3.0 mg/dL, and patients with hepatic encephalopathy were removed from the subject.

For administration of BCAA, LIVACT (registered trade mark, Ajinomoto Co., Inc.) granules having a weight ratio of isoleucine, leucine and valine of 1:2:1.2 (isoleucine: 0.952 g, leucine: 1.904 g, valine: 1.144 g) were orally administered 3 times a day after meal by one packet (4.74 g) at one time, which was continued for 2 months. Blood was drawn at the start of the administration, and 2 and 8 weeks later, and the oxidized albumin ratio (HSA(ox)%) of serum albumin was measured.

HSA(ox)% was measured as follows. Whole blood (about 4 mL) was taken and immediately centrifuged to give serum. This was passed through a 0.45 µm filter, frozen with dry ice and preserved in a deep freezer at -80°C until measurement. The sample was separated by HPLC analysis, and each area of a peak derived from reduced albumin, an oxidized albumin type I (reversible) peak and an oxidized albumin type II (irreversible) peak was calculated. HSA(ox)%= (oxidized albumin TYPE I + oxidized albumin TYPE II)/(reduced albumin + oxidized albumin TYPE I + oxidized albumin TYPE II) × 100 was employed.

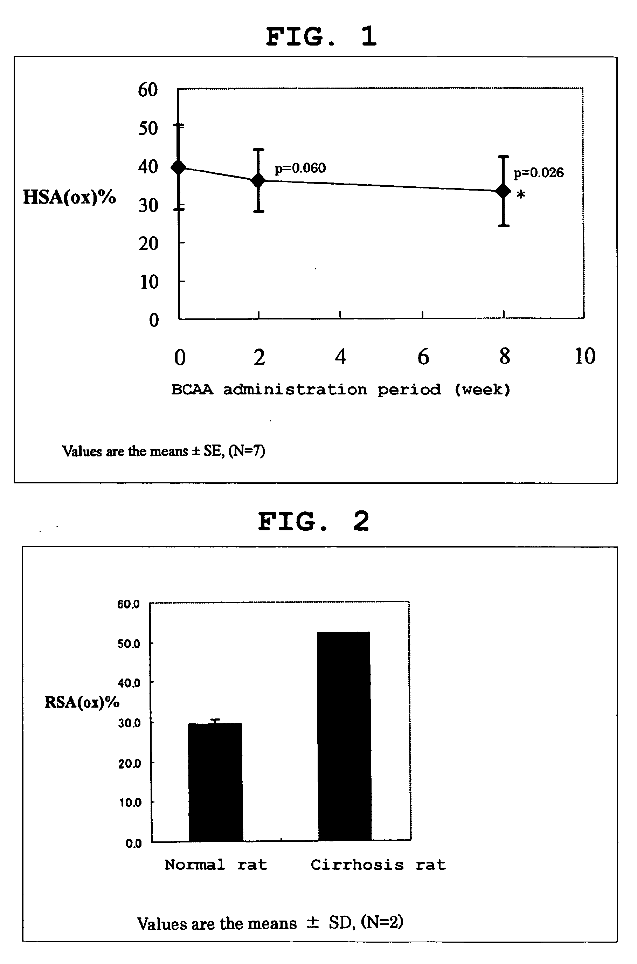

As a result, the profile of HSA(ox)% decreased over time by the administration of LIVACT as evidenced by 39.7 ± 11.1, 36.2 ± 8.1, 33.1 ± 9.0 as shown in Fig. 1, and was found to be significant 8 weeks later. At that time, the profile of the serum albumin value (g/dL) was 3.3 ± 0.2, 3.4 ± 0.2, 3.4 ± 0.3, and no significant change was observed (not shown the Figure). (*Reference relating to the measurement: Igaku-Kyoiku, Vol. 43, No. 4, pages 421 - 431 (1998))

### (Example 2)

### Analysis of oxidized albumin dysfunction in L-tryptophan binding force

For the purpose of examining the influence of an increase in the oxidized albumin ratio on the albumin function, the L-tryptophan binding rate of albumin was analyzed using plasma of cirrhosis model rats.

Generally, 90% or more of blood L-tryptophan (free-form) is bound with albumin, and albumin unbound L-tryptophan is intracerebrally converted to serotonin. Since cirrhosis patients often suffer from hepatic encephalopathy, we focused on changes in the L-tryptophan binding rate of albumin.

As a cirrhosis model rat, 25-week-old male SD rats (n=2) were prepared by administering carbon tetrachloride twice a week for 18 consecutive weeks and used, and 10-week-old healthy SD rats (n=2) were used as a control.

Under ether anesthesia, blood samples were collected from the inferior vena cava, and centrifuged using EDTA as an anticoagulant to give plasma. The plasma was preincubated at 37°C for 20 min, 10 µL of L-tryptophan solution (10 mM) was added per 1 mL of plasma and they were mixed well (A). Solution A (450 µL) was dispensed to an ultrafiltration tube (Millipore, molecular weight 30,000 cut) and centrifuged at 3,000 g for 5 min at 37°C to give 50 µL of filtrate (B) and 50 µL of unfiltrated solution (C). An equivalent amount of 8%(w/v) TCA solution was added to each of A and B, mixed and the mixture was centrifuged at room temperature and 3,000 g for 5 min. A protein-free supernatant was recovered. Thereto was added a 4-fold amount of 0.125%(v/v) heptafluorobutyric acid (HFBA) solution, mixed, diluted with 0.1% HFBA so that the concentration would fall within the range defined by the standard curve. Then, the concentration of L-tryptophan in each sample was quantified by the LC/MS method. The L-tryptophan binding rate to plasma albumin was shown by [1-(Trp concentration (B)/Trp concentration (A))]x100(%). The recovery rate was calculated by [Trp concentration (B)x50 µL + Trp concentration (C)×400 µL]/[Trp concentration (A)×450 µL]×100(%).

In addition, the oxidized albumin rate (RSA(ox)%) of solution A used then was measured and is shown in Fig. 2. The rate was 52.2 RSA(ox)% for the cirrhosis model rats, which was significantly higher as compared to 29.6 of the healthy rats.

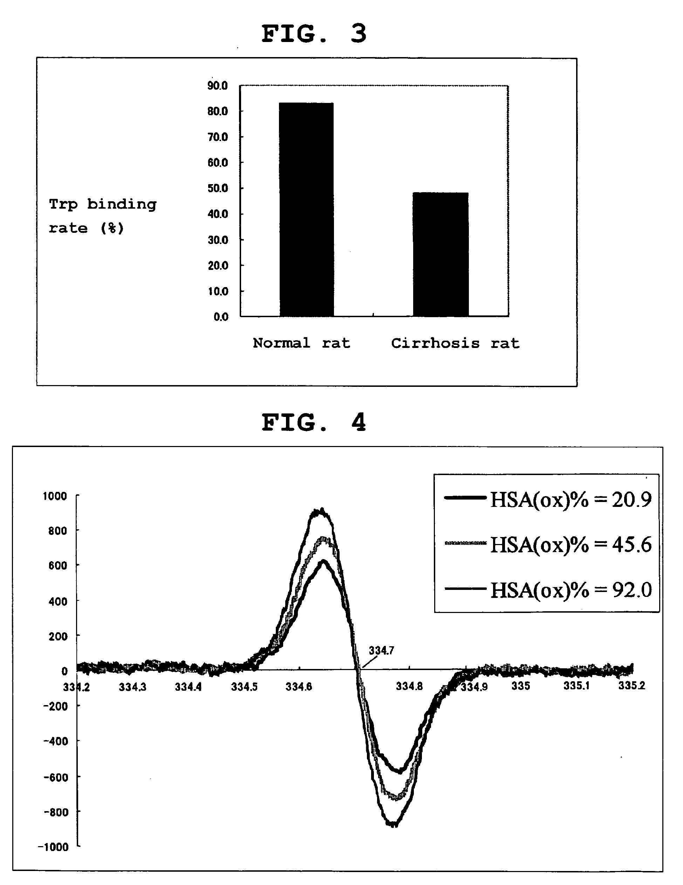

The L-tryptophan binding rate to albumin in each plasma was as high as 83% in normal rats, and decreased to 48% in cirrhosis rats (Fig. 3). Therefrom the possibility is suggested that an increase in the oxidized albumin ratio may decrease an L-tryptophan binding rate of albumin and increase the concentration of tryptophan present in blood as of a plasma protein non-binding type.

### (Example 3)

### Evaluation test of anti-oxidation performance of oxidized albumin

For the purpose of examining whether an in vivo redox state-improving effect, which is one of the functions of albumin, is influenced by an increase in the oxidized albumin ratio, the anti-oxidation performance of albumin was measured. Samples showing 3 kinds of HSA(ox)% below were prepared from albumin (HSA) purified from normal human plasma and used for the evaluation.
1. reduced albumin: purified HSA. HSA(ox)%=21.5
2. oxidized albumin: prepared by artificially forming a disulfide bond with cysteine in the 34-position cysteine residue of HSA(red). HSA(ox)%=92.0
3. post-preservation albumin: prepared by incubating plasma at 37°C for 18 hr to increase oxidized albumin ratio, followed by purification. HSA(ox)%=45.6

The anti-oxidation performance of albumin was determined by generating hydroxyl radical in vitro by a Fenton reaction and quantifying the intensity of generated radical with an ESR (Electron Spin Resonance) measurement device in the co-presence of various albumins.

25 µM FeSO₄, 5 mM DMPO (Spin Trapping Agent) and 100 µM DETAPAC (ferrous ion chelating agent) were mixed in a test tube, and various albumins were added thereto at a concentration of 10 mg/ml. Ten seconds later, 0.5 mM H₂O₂ was added and they were mixed well. The intensity of hydroxyl radical generated in 1 min from 45 sec later was measured by ESR (JES-FR80S, JEOL Ltd.). The measurement conditions were as follows.
Power: 1 mW, Modulation Width: 0.1 mT, Frequency: 100 Hz, Center Field: 335.5 mT, Receiver Gain: 79, Sweep Width: 10 mT, Time Constant: 0.03 sec, Sweep time: 1 min.

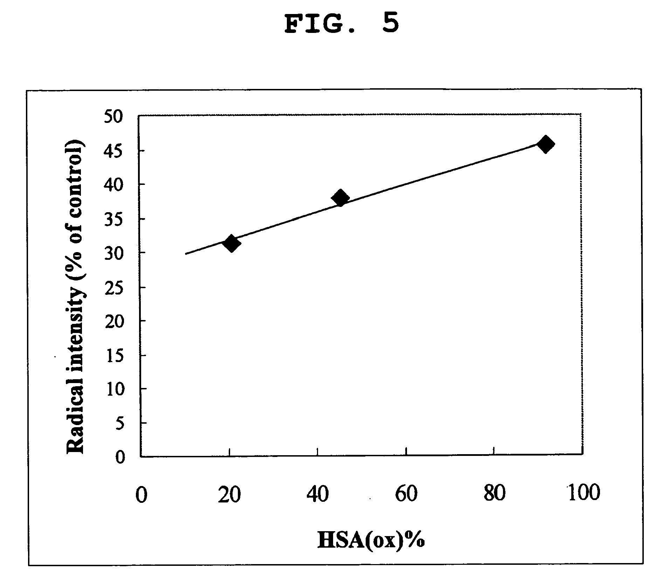

As a result, the intensity of the generated radical varied according to the kind of albumin added as shown in Fig. 4, and the obtained radical intensity was in correlation with the oxidized albumin ratio (Fig. 5). Thus, it has been shown that oxidized albumin has a weak ability to eliminate hydroxyl radical as compared to reduced albumin, and it has been suggested that an increase in the oxidized albumin ratio decreases anti-oxidation performance derived from albumin.

### Industrial Applicability

The pharmaceutical agent, food and drink provided by the present invention, which contain at least one kind of branched-chain amino acid selected from isoleucine, leucine and valine, are useful because they can improve various diseases and adverse effects caused by an increase in oxidized albumin, since they have an effect to decrease oxidized albumin, an effect to improve an oxidized/reduced albumin ratio, an effect to improve a function of serum albumin, and an effect to improve in vivo redox state. In addition, the agent for decreasing oxidized albumin of the present invention is highly safe, hardly causes side effects and is advantageous as a pharmaceutical product, food or drink because it contains amino acid as an active ingredient.

This application is based on a patent application No. 2005-226967 filed in Japan (filing date: August 4, 2005), the contents of which are incorporated in full herein by this reference.

## Claims

1. An agent for decreasing oxidized albumin, which comprises at least one kind of branched-chain amino acid selected from isoleucine, leucine and valine.

2. An agent for decreasing oxidized albumin, which comprises three kinds of branched-chain amino acid of isoleucine, leucine and valine.

3. The agent of claim 2, wherein the weight ratio of isoleucine, leucine and valine is 1:1 - 3:0.5 - 2.0.

4. The agent of claim 2, wherein the weight ratio of isoleucine, leucine and valine is 1:1.5 - 2.5:0.8 - 1.5.

5. The agent of any one of claims 1 to 4, wherein a dose of the branched-chain amino acid is 1 g - 50 g in total per day for an adult.

6. The agent of any one of claims 1 to 4, wherein the dose of the branched-chain amino acid is 3 g - 30 g in total per day for an adult.

7. The agent of any one of claims 1 to 6, which is used for· at least one kind of patient selected from patients with cirrhosis, chronic renal failure, diabetes, and under anesthesia or operation.

8. An agent for improving oxidized/reduced albumin ratio, which comprises at least one kind of branched-chain amino acid selected from isoleucine, leucine and valine.

9. An agent for improving a function of serum albumin, which comprises at least one kind of branched-chain amino acid selected from isoleucine, leucine and valine.

10. The agent of claim 9, wherein the function of serum albumin is at least one kind selected from tryptophan-binding ability, bilirubin-binding ability and fatty acid-binding ability.

11. An agent for improving a redox state in the body, which comprises at least one kind of branched-chain amino acid selected from isoleucine, leucine and valine.

12. A food or drink comprising at least one kind of branched-chain amino acid selected from isoleucine, leucine and valine, which indicates that the food or drink has an oxidized albumin lowering action.

13. A food or drink comprising at least one kind of branched-chain amino acid selected from isoleucine, leucine and valine, which indicates that the food or drink has an in vivo redox state-improving effect.

14. A method of decreasing oxidized albumin, which comprises administering an effective amount of at least one kind of branched-chain amino acid selected from isoleucine, leucine and valine to a subject.

15. A method of improving an oxidized/reduced albumin ratio, which comprises administering an effective amount of at least one kind of branched-chain amino acid selected from isoleucine, leucine and valine to a subject.

16. A method of improving a function of serum albumin, which comprises administering an effective amount of at least one kind of branched-chain amino acid selected from isoleucine, leucine and valine to a subject.

17. A method of improving a redox state in the body, which comprises administering an effective amount of at least one kind of branched-chain amino acid selected from isoleucine, leucine and valine to a subject.

18. Use of at least one kind of branched-chain amino acid selected from isoleucine, leucine and valine for the production of an agent for decreasing oxidized albumin.

19. Use of at least one kind of branched-chain amino acid selected from isoleucine, leucine and valine for the production of an agent for improving an oxidized/reduced albumin ratio.

20. Use of at least one kind of branched-chain amino acid selected from isoleucine, leucine and valine for the production of an agent for improving a function of serum albumin.

21. Use of at least one kind of branched-chain amino acid selected from isoleucine, leucine and valine for the production of an agent for improving a redox state in the body.

22. A commercial package comprising a pharmaceutical agent comprising at least one kind of branched-chain amino acid selected from isoleucine, leucine and valine and a pharmaceutically acceptable carrier, and a written matter describing that the pharmaceutical agent can or should be used for decreasing oxidized albumin.

23. A commercial package comprising a pharmaceutical agent comprising at least one kind of branched-chain amino acid selected from isoleucine, leucine and valine and a pharmaceutically acceptable carrier, and a written matter describing that the pharmaceutical agent can or should be used for improving an oxidized/reduced albumin ratio.

24. A commercial package comprising a pharmaceutical agent comprising at least one kind of branched-chain amino acid selected from isoleucine, leucine and valine and a pharmaceutically acceptable carrier, and a written matter describing that the pharmaceutical agent can or should be used for improving a function of serum albumin.

25. A commercial package comprising a pharmaceutical agent comprising at least one kind of branched-chain amino acid selected from isoleucine, leucine and valine and a pharmaceutically acceptable carrier, and a written matter describing that the pharmaceutical agent can or should be used for improving a redox state in the body.

26. A composition for decreasing oxidized albumin, which comprises at least one kind of branched-chain amino acid selected from isoleucine, leucine and valine.

27. A composition for improving an oxidized/reduced albumin ratio, which comprises at least one kind of branched-chain amino acid selected from isoleucine, leucine and valine.

28. A composition for improving a function of serum albumin, which comprises at least one kind of branched-chain amino acid selected from isoleucine, leucine and valine.

29. A composition for improving a redox state in the body, which comprises at least one kind of branched-chain amino acid selected from isoleucine, leucine and valine.
